**Europäisches Patentamt**

(19) **European Patent Office**

**Office européen des brevets**

(11) Veröffentlichungsnummer: **0 010 621**
**A1**

(12) **EUROPÄISCHE PATENTANMELDUNG**

(21) Anmeldenummer: 79103692.4

(22) Anmeldetag: 28.09.79

(51) Int. Cl.³: **A 61 M 25/00, C 08 J 7/00**

(30) Priorität: 06.10.78 CH 10439/78

(71) Anmelder: **Intermedicat GmbH, Gerliswilstrasse 45, CH-6020 Emmenbrücke (CH)**

(43) Veröffentlichungstag der Anmeldung: 14.05.80
**Patentblatt 80/10**

(72) Erfinder: **Braun, Bernd, Dr., Tränkelücke 1, D-3508 Melsungen (DE)**

(84) Benannte Vertragsstaaten: **AT BE CH DE FR GB IT LU NL SE**

(74) Vertreter: **von Kreisler, Alek, Dipl.-Chem. et al, Deichmannhaus am Hauptbahnhof, D-5000 Köln 1 (DE)**

(54) Verfahren zur Herstellung eines Kunststoffverweilkatheters mit negativ geladener Oberfläche und danach hergestellter Verweilkatheter.

(57) Ein antithrombogener Verweilkatheter mit negativ aufgeladener Oberfläche wird hergestellt, indem man einen Katheter durch mindestens einmaliges Eintauchen in eine Pyridin- oder Toluol/Tetrahydrofuranlösung eines Copolymerisates des Äthylens mit Acrylsäure, deren Estern oder Salzen mit einem negativ aufgeladenen Überzug versieht und nach dem letzten Eintauchen den Überzug bis zur Lösungsmittelfreiheit trocknet.

EP 0 010 621 A1

- 1 -

Verfahren zur Herstellung eines Kunststoffverweilkatheters
mit negativ geladener Oberfläche

---

Es ist bekannt, daß Verweilkatheter aus Polyvinylchlorid,
Polyäthylen mit niedriger verfügbarer Elektronendichte
und Kunststoffe mit positiv geladenen Molekülgruppen eine
besonders starke Wechselwirkung zu Blutbestandteilen mit
elektronegativer (Oberflächen)-ladung haben. Die besonders
deutliche Wechselwirkung zwischen positiv geladenen oder
neutralen Materialoberflächen und Blutplättchen führt über
einen komplizierten Gerinnungsmechanismus - wie durch
rasterelektronenmikroskopische Aufnahmen gezeigt werden
kann, zur Thrombenbildung. Bei entsprechender Größe können
die Blutgerinsel und Thromben abgelöst werden, in den Blutkreislauf gelangen und in Lunge und Gehirn zu lebensbedrohenden
Thrombosen führen.

Aus der Literatur, z.B. Sawyer, Trans. Amer. Soc. Artif. Organs,
Volume XVI, S. 1, 1970, ist weiterhin bekannt, daß einige Kunststoffe, vornehmlich solche mit negativ geladenen Molekülgruppen
wie Äthylen-Acrylsäurecopolymerisate (EAA) und ihre Natriumsalze,
Kunststoffe, die Crotonsäure als Copolymeres enthalten, weiterhin sulfonierte Kunststoffe, die $SO_3H$-Gruppen enthalten und
Kunststoffe, die mit dem $SO_3H$-gruppenhaltigen Heparin beschichtet sind, eine elektronegative Aufladung zeigen und daß
Katheter, die aus diesen Kunststoffen hergestellt sind, die
obigen pathologischen Erscheinungen der Thrombosegefahr nicht
zeigen.

Eine geeignete Methode zur Festlegung der Elektronegativität einzelner Kunststoffoberflächen ist die Bestimmung des Zeta Potentials.

Diese Polymeren haben jedoch den großen Nachteil, daß sie unter den für Thermoplaste üblichen Verarbeitungsbedingungen außerordentlich stark an den die Kunststoffschmelze führenden Verarbeitungseinrichtungen kleben und dadurch das Extrudieren von feinen, für Katheter geeignete Schläuche von 0,5 - 6 mm Durchmesser sehr erschwert bis unmöglich ist.

Die mit vielen Schwierigkeiten so hergestellten, kaum maßhaltigen und ungleich geformten Schläuche zeigen zwar im Kontakt mit Blut die guten Eigenschaften der oben erwähnten, mit negativem Potential aufgeladenen Materialien, sie sind aber praktisch für eine Fabrikation und in der klinischen Anwendung auch wegen ihrer relativ hohen Härte und Steifigkeit unbrauchbar. Um Verletzungen einer Gefäßwand beim Einführen des Katheters zu vermeiden, werden üblicherweise Kathetermaterialien gefordert, die eine Shore Härte A von 50 - 60 aufweisen, während z.B. Katheter aus Äthylen-Acrylsäurecopolymerisat Shore Härten D von 50 - 60 haben.

Eigene und in der Praxis durchgeführte Versuche haben zu dem überraschenden Ergebnis geführt, daß es möglich ist, die bisher verwendeten Polyvinylchloridweich (PVC)-und Polyäthylen-(PE)Katheter mit einer dünnen Schicht eines Äthylen-Acrylsäurecopolymerisats (EAA), oder einem Ester oder einem Salz des Äthylen-Acrylsäurecopolymerisats zu beschichten. Dadurch ist es möglich, die gewünschte antithrombogenen Eigenschaften eines Verweilkatheters zu erreichen und die Gefahr der Thrombose beim Katheterisieren zu verringern.

Die dünne Schicht kann als einzelne Schicht oder eine Mehrzahl von Schichten aufgebracht werden. Der Katheter sollte
zunächst gereinigt werden, um die Schmiermittel auf seiner
Oberfläche zu entfernen.

Die dünne Schicht oder der Überzug kann in geeigneter Weise
durch Verwendung einer Lösung des Äthylen-Acrylsäurecopolymerisats oder eines Esters oder eines Salzes des Copolymerisats in einem Lösungsmittel aufgebracht/Die Lösung kann beispielsweise durch Tauchen des Katheters in die Lösung oder
durch andere geeignete Methoden wie·z.B. Besprühen oder
Bespritzen aufgebracht/Geeignete Lösungsmittel für das Copolymerisat sind Pyridin oder ein Gemisch von Toluol und
Tetrahydrofuran. Andere Lösungsmittel oder Lösungsmittelgemische können auch verwendet werden.

Nachdem die Lösung des Copolymerisats aufgebracht worden ist,
wird der Katheter erwärmt, um die Überzugsschicht zu trocknen.
Wenn zwei oder mehrere Schichten aufgebracht werden, sollte
die vorhergehende Schicht nur so weit getrocknet werden,bis
sieklebrig ist, worauf dann die nachfolgende Schicht aufgetragen werden sollte. Nachdem alle Schichten aufgebracht
wordensind, wird der Überzug getrocknet, bis keine nachweisbare Menge an Lösungsmittel aus dem Überzug freigegeben wird.

Der fertig beschichtete Katheter kann dann für die Verwendung
sterilisiert werden.

Das folgende Beispiel erläutert die Erfindung.

Beispiel:

Die gut von Schmiermitteln befreiten PVC-und PE-Katheter
werden in eine 1%ige Lösung von EAA in Toluol/Tetrahydrofuran 1:1 getaucht und im Wärmekanal bei 40 $^{\circ}$C leicht
getrocknet.

- 4 -

Im noch klebrigen Zustand taucht man nochmals kurz
in eine Lösung von 0,1 % EAA in Toluol/Tetrahydrofuran 1 : 1 und trocknet anschließend im Wärmekanal
bei etwa 60 $^{O}$C solange, bis alle Lösungsmittel verdampft, chemisch und physiologisch nicht mehr nachweisbar und die Katheteroberfläche vollkommen trocken
ist.

In einer entsprechenden Einmal-Steril-Verpackung werden
sie durch Gammabestrahlung mit etwa 2,5 Mrad für die
klinische Anwendung sterilisiert. Die so behandelten,
in den Blutkreislauf implantierten Katheter zeigen im
Tier-und Klinik-Versuch die gewünschten antithrombogenen
Eigenschaften und schließen die Gefahr der Thrombose
bei der Katheterisierung weitgehend aus.

Patentansprüche

1. Verfahren zur Herstellung eines antithrombogenen Verweilkatheters mit negativ aufgeladener Oberfläche, dadurch
   gekennzeichnet, dass man einen Katheter durch mindestens
   einmaliges Eintauchen in eine/Toluol/Tetrahydrofuranlö-
                                  Pyridin oder
   sung eines Copolymerisates des Aethylens mit Acrylsäure,
   deren Estern oder Salzen mit einem negativ aufgeladenen
   Ueberzug versieht und nach dem letzten Eintauchen den
   Ueberzug bis zur Lösungsmittelfreiheit trocknet.

2. Verfahren nach Patentanspruch 1, dadurch gekennzeichnet,
   dass man einen Katheter aus Polyvinylchlorid oder Polyäthylen verwendet.

3. Verfahren nach Patentanspruch 2, dadurch gekennzeichnet,
   dass man den Katheter vor dem Eintauchen von Schmiermitteln befreit.

4. Verfahren nach Patentanspruch 1, dadurch gekennzeichnet,
   dass man nach dem ersten Eintauchen nur antrocknet und in
   noch klebrigem Zustand noch einmal eintaucht.

5. Antithrombogener Verweilkatheter mit negativ aufgeladener
   Oberfläche, hergestellt nach dem Verfahren der Ansprüche 1 - 4.

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - A1 - 2 622 394 (MEADOX MEDICALS INC.) <br> * Ansprüche 1, 2; Seiten 2, 4 * <br> -- | 1 |
| | US - A - 3 695 921 (T.H. SHEPHERD et al.) <br> * Anspruch 1 * <br> -- | 1,2 |
| A | DE - B2 - 2 140 994 (SIEMENS AG.) <br> * ganzes Dokument * <br> -- | |
| A | DE - A - 2 324 320 (THE UPJOHN CO.) <br> * ganzes Dokument * <br> ---- | |

**KLASSIFIKATION DER ANMELDUNG (Int.Cl.3)**

A 61 M 25/00

C 08 J 7/00

**RECHERCHIERTE SACHGEBIETE (Int. Cl.3)**

A 61 M 5/00

A 61 M 25/00

B 05 D 1/00

C 08 J 7/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 29-01-1980 | SCHLAITZ |

EPA form 1503.1 06.78